(19)

Europäisches
Patentamt

European
Patent Office

Office européen
des brevets

(11) **EP 4 481 158 B1**

(12) **EUROPEAN PATENT SPECIFICATION**

(45) Date of publication and mention
of the grant of the patent:
**05.08.2026 Bulletin 2026/32**

(21) Application number: **24183628.7**

(22) Date of filing: **21.06.2024**

(51) International Patent Classification (IPC):
***E21B 43/16*** (2006.01)

(52) Cooperative Patent Classification (CPC):
**E21B 43/16; E21B 2200/20**

(54) **ASPHALTENE SIMULATION USING A PSEUDO-COMPONENT FRAMEWORK**

ASPHALTENSIMULATION MIT EINEM PSEUDOKOMPONENTENRAHMENWERK

SIMULATION D'ASPHALTÈNES À L'AIDE D'UNE STRUCTURE PSEUDO-COMPOSANT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **23.06.2023 US 202318340492**

(43) Date of publication of application:
**25.12.2024 Bulletin 2024/52**

(73) Proprietors:
• **Chevron U.S.A. Inc.
San Ramon, CA 94583 (US)**
• **Schlumberger Technology Corporation
Sugar Land, Texas 77478 (US)**
• **TotalEnergies OneTech
92400 Courbevoie (FR)**

(72) Inventors:
• **SHI, Xundan
94583 San Ramon (US)**
• **CHANG, Yih-Bor
94583 San Ramon (US)**
• **WOLFSTEINER, Christian
94583 San Ramon (US)**
• **HAN, Choongyong
94583 San Ramon (US)**
• **GUYAGULER, Baris
94583 San Ramon (US)**

(74) Representative: **Haseltine Lake Kempner LLP
One Portwall Square
Portwall Lane
Bristol BS1 6BH (GB)**

(56) References cited:
• **KHANIFAR AHMAD ET AL: "Modelling of
Asphaltene Precipitation and Deposition during
WAG Application", INTERNATIONAL
PETROLEUM TECHNOLOGY CONFERENCE, 7
February 2012 (2012-02-07), Bangkok, Thailand,
XP093219529, Retrieved from the Internet
<URL:https://watermark.silverchair.com/
iptc-14147-ms.pdf?
token=AQECAHi208BE49Ooan9kkhW_Er
cy7Dm3ZL_9Cf3qfKAc485ysgAAA3owggN2Bgk
qhkiG9w0BBwagggNnMIIDYwIBADCCA1wGCSq
GSIb3DQEHATAeBglghkgBZQMEAS4wEQQMB
l3OrVsBUmX-XySpAgEQgIIDLU1uu1h32zwMFG
o1wSHUoydE6h1f4HBD96T983U8PoEipK0rZqKJ
NitBfLyDRT_lwpuSeKTrTfuk__ieM6Z_eP>
[retrieved on 20241030]**
• **AHMAD KHANIFAR ET AL: "Study of asphaltene
precipitation and deposition phenomenon",
NATIONAL POSTGRADUATE CONFERENCE
(NPC), 2011, IEEE, 19 September 2011
(2011-09-19), pages 1 - 6, XP032099774, ISBN:
978-1-4577-1882-3, DOI: 10.1109/
NATPC.2011.6136532**

EP 4 481 158 B1

**Description**

*FIELD*

The present disclosure relates generally to the field of asphaltene simulation using a pseudo-component framework.

*BACKGROUND*

Asphaltene precipitation, flocculation, and deposition in a reservoir may result in permeability damage and/or viscosity reduction in the reservoir, which may negatively impact production from the reservoir. Modeling asphaltene using chemical reactions may result in a numerical system that is not stable and is computationally intensive to solve.

Reference may be made to "Modelling of Asphaltene Precipitation and Deposition during WAG Application" by Khanifar Ahmad et al, International Petroleum Technology Conference, 2012-02-07, 20120207 - 20120209, Bangkok, Thailand.

Reference may also be made to "Study of asphaltene precipitation and deposition phenomenon" by Khanifar Ahmad et al, National Postgraduate Conference, 2011.

*SUMMARY*

This disclosure relates to performing asphaltene simulation using a pseudo-component framework. The present invention is defined by the appended independent claims, to which reference may now be made. Specific embodiments are defined by the dependent claims.

These and other objects, features, and characteristics of the system and/or method disclosed herein, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a," "an," and "the" include plural referents unless the context clearly dictates otherwise.

*BRIEF DESCRIPTION OF THE DRAWINGS*

FIG. 1 illustrates an example system for asphaltene simulation.
FIG. 2 illustrates an example method for asphaltene simulation.
FIG. 3 illustrates asphaltene precipitation, flocculation, and deposition.
FIG. 4 illustrates an example listing of pseudo components.
FIG. 5 illustrates an example flow diagram for asphaltene simulation.

*DETAILED DESCRIPTION*

The present disclosure relates to performing asphaltene simulation using a pseudo-component framework. Asphaltene simulation is performed by modeling asphaltene using physical changes. A pseudo-component framework is used to simulate asphaltene precipitation, asphaltene flocculation, and asphaltene deposition in a subsurface region. The pseudo-component framework for asphaltene simulation treats asphaltene precipitation, asphaltene flocculation, and asphaltene deposition as physical changes of a single component, rather than as chemical changes. Use of the pseudo-component framework for asphaltene simulation reduces complexity of asphaltene simulation. For example, use of the pseudo-component framework for asphaltene simulation enables tracking of asphaltene as it is found in different states (precipitated, flocculated, deposited). Use of the pseudo-component framework for asphaltene simulation enables chemical reactions to be replaced by a flash and adsorption framework. Asphaltene simulation using the pseudo-component framework exhibits stable and fast convergence.

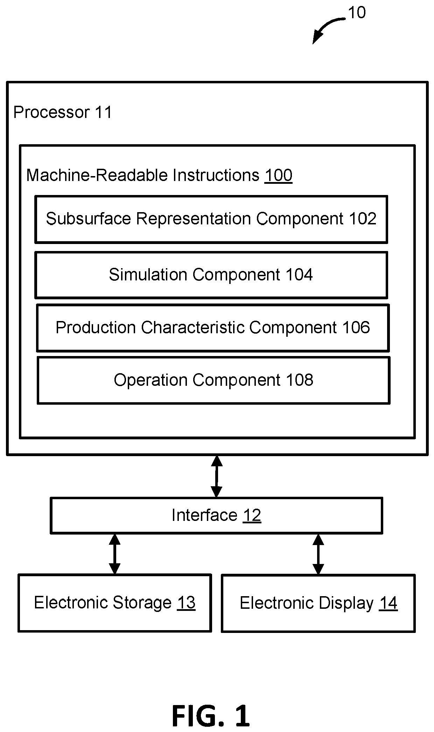

The methods and systems of the present disclosure may be implemented by a system and/or in a system, such as a system 10 shown in FIG. 1. The system 10 may include one or more of a processor 11, an interface 12 (e.g., bus, wireless interface), an electronic storage 13, an electronic display 14, and/or other components. Subsurface representation information and/or other information may be obtained by the processor 11. The subsurface representation information may define a subsurface representation. The subsurface representation may define subsurface configuration of a subsurface region. Asphaltene precipitation, asphaltene flocculation, and asphaltene deposition in the subsurface region may be simulated by the processor 11 based on the subsurface representation, a pseudo-component framework for asphaltene simulation, and/or other information. The pseudo-component framework for asphaltene simulation may treat the asphaltene precipitation, the asphaltene flocculation, and the asphaltene deposition as physical changes of a single component.

One or more production characteristics of the subsurface region may be determined by the processor 11 based on the simulation of the asphaltene precipitation, the asphaltene flocculation, and the asphaltene deposition in the subsurface region, and/or other information. One or more operations for the subsurface region may be facilitated by the processor 11 based on the production characteristic(s) of the subsurface region and/or other information.

The electronic storage 13 may be configured to include one or more electronic storage media that electronically stores information. The electronic storage 13 may store software algorithms, information determined by the processor 11, information received remotely, and/or other information that enables the system 10 to function properly. For example, the electronic storage 13 may store information relating to a subsurface region, subsurface representation information, information relating to a subsurface representation, information relating to subsurface configuration, information relating to asphaltene, information relating to asphaltene precipitation, information relating to asphaltene flocculation, information relating to asphaltene deposition, information relating to a pseudo-component framework, information relating to physical changes of a single component, information relating to production characteristics, information relating to operations, and/or other information.

The electronic display 14 may refer to an electronic device that provides visual presentation of information. The electronic display 14 may include a color display and/or a non-color display. The electronic display 14 may be configured to visually present information. The electronic display 14 may present information using/within one or more graphical user interfaces. For example, the electronic display 14 may present information relating to a subsurface region, subsurface representation information, information relating to a subsurface representation, information relating to subsurface configuration, information relating to asphaltene, information relating to asphaltene precipitation, information relating to asphaltene flocculation, information relating to asphaltene deposition, information relating to a pseudo-component framework, information relating to physical changes of a single component, information relating to production characteristics, information relating to operations, and/or other information.

Asphaltene may refer to molecular substances found in oil (e.g., crude oil). Asphaltene may refer to organic materials including aromatic and naphthenic ring compounds. Asphaltene may be made up of carbon, hydrogen, nitrogen, oxygen, and/or other elements. Asphaltene precipitation, flocculation, and deposition may occur in a subsurface region (e.g., a reservoir), subsurface equipment (e.g., a well, equipment in a well such as tubing, etc.), and/or surface equipment (e.g., equipment in a surface facility such as conduits, separators, etc.) during production of resources, such as oil or hydrocarbon, from the subsurface region. Asphaltene precipitation, flocculation, and deposition in a subsurface region may cause plugging in the subsurface region and/or the equipment for the subsurface region, which may negatively impact production from the subsurface region.

Accurate modeling of asphaltene precipitation, flocculation, and deposition in a subsurface region may improve production of resources from the subsurface region. Accurate modeling of asphaltene precipitation, flocculation, and deposition may enable determination of how the characteristics of the subsurface region are changing. For example, accurate modeling of asphaltene precipitation, flocculation, and deposition may enable changes in permeability and/or viscosity in the subsurface region to be determined. Understanding changes in the characteristics of the subsurface region may enable operators to take actions to improve operations in the subsurface region, such as to increase production from the subsurface region and/or to increase the efficiency of operations in the subsurface region.

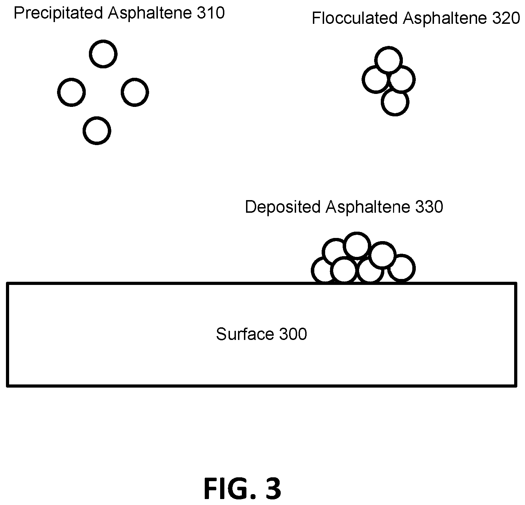

FIG. 3 illustrates example asphaltene precipitation, flocculation, and deposition. Asphaltene may precipitate from oil, resulting in precipitated asphaltene 310. Asphaltene precipitation may be caused by a number of factors, such as changes in pressure, temperature, and/or composition. The precipitated asphaltene 310 may aggregate/cluster together and form flocculated asphaltene 320. The flocculated asphaltene 330 may attach/stick to a surface 300, such as a surface in a well (e.g., tubing in a well) and/or a surface in a subsurface region (e.g., a rock matrix in a subsurface region).

Changes in asphaltene, such as asphaltene precipitation and flocculation, may be modeled using a chemical-reaction model. For example, changes between precipitation and flocculation of asphaltene may be modeled as chemical reactions that leads to chemical transformation of asphaltene. However, such modeling of asphaltene may result in a numerical system that is not stable and is computationally intensive to solve.

The present disclosure provides a tool to accurately model asphaltene precipitation, flocculation, and deposition in a subsurface region using a pseudo-component framework. Rather than modeling changes in asphaltene as chemical changes, the pseudo-component framework treats asphaltene precipitation, asphaltene flocculation, and asphaltene deposition as physical changes of a single component. The pseudo-component framework treating asphaltene precipitation, asphaltene flocculation, and asphaltene deposition as physical changes of a single component may include the pseudo-component framework treating precipitated asphaltene, flocculated asphaltene, and deposited asphaltene as same component (e.g., same molecules) in different physical form. The pseudo-component framework treating asphaltene precipitation, asphaltene flocculation, and asphaltene deposition as physical changes of a single component may include the pseudo-component framework treating changes between precipitated asphaltene and flocculated asphaltene as physical changes (change in form) of the same component.

Modeling of asphaltene using the pseudo-component framework may be more stable and efficient, and computationally less expensive than modeling of asphaltene using a chemical-reaction framework. Numerical stability and computational efficiency may be achieved via use of the pseudo-component framework. Chemical reactions, which results in extra material balance equations and thereby increases the size of the linear system, may be replaced by a flash and adsorption framework, which include secondary equations that are algebraically "reduced" away and do not add to size of linear system. The pseudo-component framework enables this by tracking different states of asphaltene (e.g., precipitated, flocculated, deposited) as pseudo-components of the same component.

Modeling of asphaltene provided by the present disclosure enables operations in the surface region to be improved. For example, modeling of asphaltene using the pseudo-component framework may be used to design operations (e.g., well operations) to reduce asphaltene precipitation, asphaltene flocculation, and/or asphaltene deposition in the subsurface region, which may reduce permeability damage and/or viscosity reduction in the subsurface region. Modeling of asphaltene using the pseudo-component framework may be used to simulate operations to reduce/minimize impact of asphaltene to subsurface region operations (e.g., perform simulation of whether particular solvent will dissolve asphaltene).

The pseudo-component framework treating asphaltene precipitation, asphaltene flocculation, and asphaltene deposition as physical changes of a single component may include the pseudo-component framework tracking the amounts of asphaltene precipitation and asphaltene flocculation as pseudo-components of the same component and tracking the amounts of asphaltene deposition using adsorption. The pseudo-component framework treating asphaltene precipitation, asphaltene flocculation, and asphaltene deposition as physical changes of a single component may include the pseudo-component framework tracking the amounts of asphaltene precipitation, asphaltene flocculation, and asphaltene deposition without the use of chemical reactions.

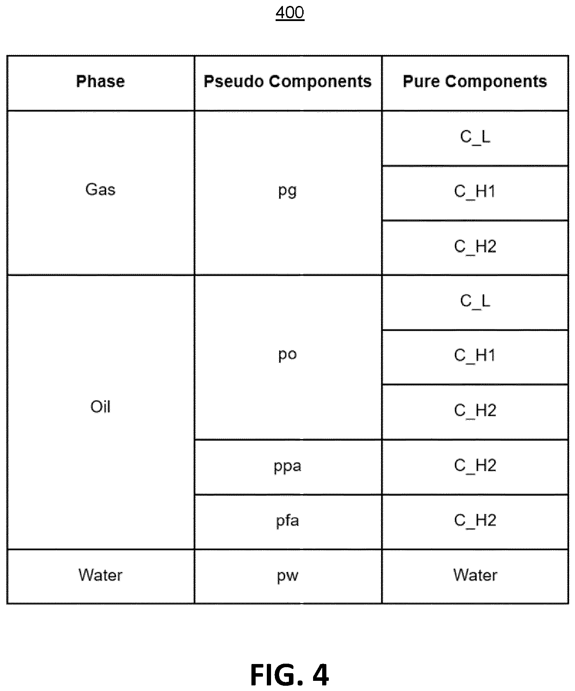

FIG. 4 illustrates an example listing of pseudo components in different phases 400 within the pseudo-component framework. Within the pseudo-component framework, various components in the subsurface region may be grouped as pseudo components in a particular phase. The pseudo components of a phase may flow with the same speed of that phase. In oil phase, precipitated asphaltene and flocculated asphaltene may flow with oil. The amount (e.g., volume) of precipitated asphaltene and flocculated asphaltene may be tracked as pseudo component amount (e.g., volume) in the oil phase.

The listing 400 may show components in gas phase, oil phase, and water phase. The gas phase and the water phase may each include a single pseudo component: a pseudo gas (pg) component in the gas phase and a pseudo water (pw) component in the water phase. The pseudo gas (pg) component may include three hydrocarbon components: one light hydrocarbon component (C_L) and two heavy hydrocarbon components (C_H1, C_H2). Other numbers of hydrocarbon components are contemplated. The pseudo water (pw) component may include water.

The oil phase may include a pseudo oil (po) component, a pseudo precipitated asphaltene (ppa) component, and a pseudo flocculated asphaltene (pfa) component. The oil phase may not include a pseudo component for deposited asphaltene as deposited asphaltene generally sticks to the surface (e.g., of well tubing, of rock) and will not move with the oil. The deposited asphaltene may be modeled with adsorption.

In the oil phase, precipitated asphaltene and flocculated asphaltene may be treated as two separate pseudo components. Using the example of three hydrocarbons, the pseudo oil (po) component may include the three hydrocarbons (one light and two heavy). The pseudo oil (po) component may include hydrocarbon components that have not changed into precipitated asphaltene or flocculated asphaltene. The pseudo precipitated asphaltene (ppa) component and the pseudo flocculated asphaltene (pfa) component may include the heaviest hydrocarbon (C_H2).

As pressure drops, the heaviest hydrocarbon may be precipitated from the oil phase and become small solid particles. Some of the small solid particles may aggregate/cluster together to form flocculated asphaltene. Heavy hydrocarbons may refer to hydrocarbons that have enough mass that they aggregate/cluster together to form precipitated or flocculated asphaltene. Light hydrocarbons may refer to hydrocarbons that do not have enough mass that they do not aggregate/cluster together to form precipitated or flocculated asphaltene. As shown in the listing 400, the pseudo precipitated asphaltene (ppa) component and the pseudo flocculated asphaltene (pfa) component may include the same pure component (C_H2). Rather than treating them as different components, the pseudo-component framework may treat the pseudo precipitated asphaltene (ppa) component and the pseudo flocculated asphaltene (pfa) component as different physical forms of the same component.

The volume of precipitated asphaltene in oil phase may be represented by volumetric concentration of pseudo precipitated asphaltene (ppa) component: $C_{O,ppa}$. The volume of flocculated asphaltene in oil phase may be represented by volumetric concentration of pseudo flocculated asphaltene (pfa) component: $C_{O,pfa}$. The volumetric concentration of a pseudo component $C_{\alpha,pc}$ may be defined as the volume ratio of a pseudo component over the phase volume. Given that the sum of the volume of pseudo precipitated asphaltene (ppa) component, pseudo flocculated asphaltene (pfa) component, and pseudo oil (po) component equals the oil phase volume, the following normalized relationship may be established, where $C_{O,po}$ is pseudo oil (po) component volumetric concentration in oil phase:

$$C_{O,ppa} + C_{O,pfa} + C_{O,po} = 1 \tag{1}$$

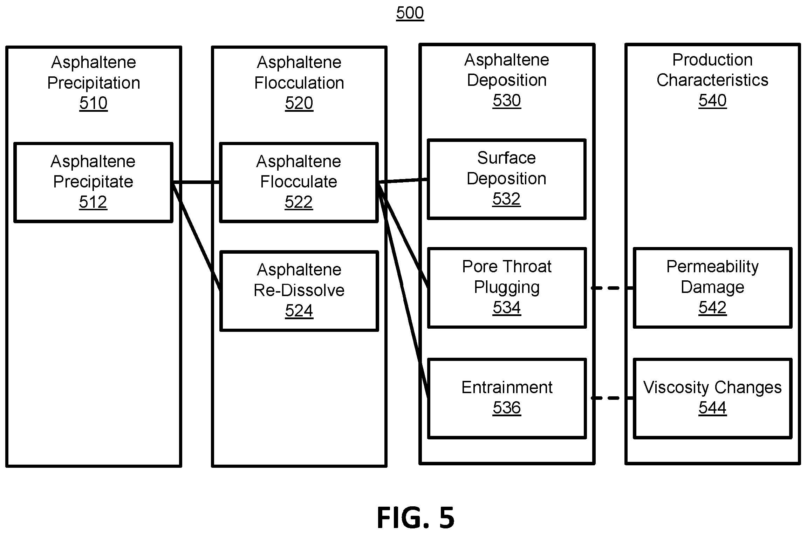

FIG. 5 illustrates an example flow diagram for asphaltene simulation 500. Asphaltene simulation 500 may be performed for a subsurface region. At step 510, asphaltene precipitation from oil may be modeled. Whether asphaltene will precipitate from oil and/or how much asphaltene will precipitate from oil may be determined. The oil components may be split into a non-precipitating part in pseudo oil (po) component and a precipitating part in pseudo asphaltene (ppa) component. The precipitated asphaltene may be referred to as asphaltene precipitate 512. In some implementations, asphaltene precipitation from oil may be determined based on fugacity, pressure, temperature, and/or other factors. For example, asphaltene precipitation from oil may be modeled using thermodynamic equilibrium using fugacity below, where $f_{po,h}$ is fugacity of heavy component in pseudo oil (po), $f_{ppa}$ is precipitated asphaltene fugacity, $f_{ppa}^*$ is the precipitated asphaltene fugacity at reference pressure $p^*$, $p$ is pressure, $v_{ppa}$ is the precipitated asphaltene molar volume, R is ideal gas constant, and T is temperature:

$$\ln f_{po,h} = \ln f_{ppa} \tag{2}$$

$$\ln f_{ppa} = \ln f_{ppa}^* + \frac{v_{ppa}(p-p^*)}{RT} \tag{3}$$

With the precipitation of asphaltene governed (e.g., modeled) by the above thermodynamic equilibrium condition, asphaltene precipitation may be reversible. Precipitated asphaltene may return to fluid state when the conditions return to a state outside the asphaltene precipitation envelope. Whether asphaltene precipitation occurs may be determined based on a stability test. Asphaltene precipitation may occur if fugacity of heavy component in pseudo oil ($f_{po,h}$) is greater than or equal to precipitated asphaltene fugacity ($f_{ppa}$). Asphaltene precipitation may not occur if fugacity of heavy component in pseudo oil ($f_{po,h}$) is less than precipitated asphaltene fugacity ($f_{ppa}$). When asphaltene precipitation occurs, the amount of precipitated asphaltene may be determined by solving the following equilibrium equation:

$$f_{po,h} = f_{ppa} \tag{4}$$

At step 520, asphaltene flocculation from the asphaltene precipitate 512 may be modeled. How much of the asphaltene precipitate 512 will flocculate may be determined. Asphaltene flocculation may be a time dependent process. Aggregation of smaller asphaltene precipitate 512 may result in formation of asphaltene flocculate 522. At the same time, the asphaltene flocculate 522 may break up into smaller particles. This process may be referred to as asphaltene re-dissolve 524. Thus, asphaltene flocculation may include a forward aggregation of asphaltene flocculate 522 and a backward disaggregation of asphaltene re-dissolve 524. The forward aggregation of asphaltene and backward disaggregation of asphaltene may reach equilibrium.

Aggregation and disaggregation of asphaltene flocculate 522 may be treated as a physical change, rather than as a chemical reaction. The forward aggregation of asphaltene flocculate 522 may be governed by the below, where $\rho_{pfa}$ is molar density for pseudo flocculated asphaltene (pfa) component, $\rho_{ppa}$ is molar density for pseudo precipitated asphaltene (ppa) component, and $k_{af}$ is forward rate formation of flocculated asphaltene (asphaltene flocculate 522) from precipitated asphaltene (asphaltene precipitate 512):

$$\frac{\partial(\rho_{pfa}C_{O,pfa})}{\partial t} = k_{af}\rho_{ppa}C_{O,ppa} \tag{5}$$

The backward disaggregation of asphaltene flocculate 522 (asphaltene re-dissolve 524) may be governed by the below, where $k_{fa}$ is backward rate formation of precipitated asphaltene (asphaltene precipitate 512) from flocculated asphaltene (asphaltene flocculate 522):

$$\frac{\partial(\rho_{pfa}C_{O,pfa})}{\partial t} = -k_{fa}\rho_{pfa}C_{O,pfa} \tag{6}$$

The forward aggregation equation (5) and the backward disaggregation equation (6) may be combined to obtain the following net flocculate rate equation:

$$\frac{\partial(\rho_{pfa}C_{O,pfa})}{\partial t} = k_{af}\rho_{ppa}C_{O,ppa} - k_{fa}\rho_{pfa}C_{O,pfa} \tag{7}$$

Asphaltene flocculate 522 may disaggregate back to asphaltene precipitate 512, and then return to fluid state when the conditions return to a state outside the asphaltene precipitation envelope. If the backward rate formation of precipitated asphaltene ($k_{fa}$) is zero, then the flocculation process may be irreversible. The forward rate formation of flocculated asphaltene ($k_{af}$) and the backward rate formation of precipitated asphaltene ($k_{fa}$) may be user-controlled parameters. These parameters may be set/adjusted to customize modeling of time-dependent flocculation.

At step 530, asphaltene deposition may be modeled. An adsorption process may be used to model asphaltene deposition. Asphaltene deposition may include the asphaltene flocculate 522 attaching/sticking to a surface, such as a surface in a well or a surface in a subsurface region. When the asphaltene flocculate 522 grows to a certain point, it may no longer flow with the oil phase. At this point, the asphaltene flocculate 522 may attach/stick to the surface via surface deposition 532, pore throat plugging 534, and/or entrainment 536. The surface deposition 532 may govern the rate at which asphaltene flocculate 522 deposits on the surface. The pore throat plugging 534 may govern the rate at which asphaltene flocculate 522 clogs up pores (e.g., pores in the subsurface region). Entrainment 536 may govern the rate at which asphaltene is carried away from the surface/pores by fluid (e.g., oil). The rate of asphaltene deposition ( $\frac{\partial V_{s2}^d}{\partial t}$ ) may be governed by the following, with the first term representing surface deposition 532, the second term representing pore throat plugging 534, and the third term representing entrainment 536:

$$\frac{\partial V_{s2}^d}{\partial t} = \alpha\rho_{pfa}C_{O,pfa}\emptyset + \gamma u_o\rho_{pfa}C_{O,pfa}\emptyset - \beta V_{s2}^d(v_0 - v_{cr}) \tag{8}$$

In the first term for surface deposition 532, $\alpha$ may be a surface deposition rate coefficient and $\emptyset$ may be porosity. The surface deposition rate coefficient and/or other parameters may be user-controlled parameter(s). In the second term for pore throat plugging 534, $\gamma$ may be a pore throat plugging rate coefficient and $u_o$ may be oil phase Darcy velocity. The pore throat plugging rate coefficient and/or other parameters may be user-controlled parameter(s). The rate of pore throat plugging may be directly proportional to the product of oil phase Darcy velocity ($u_o$), which may be defined by the following, where $\mu_o$ is oil phase viscosity and k is absolute permeability:

$$u_O = -\frac{k}{\mu_O}\max\left(\frac{\partial P}{\partial X}, \frac{\partial P}{\partial Y}, \frac{\partial P}{\partial Z}\right) \tag{9}$$

The pore throat plugging rate coefficient (y) may be defined by the following, where $\gamma_i$ is the instantaneous pore throat plugging rate coefficient , $V_{s2}^d$ is amount of asphaltene deposits/fractional pore volume occupied by asphaltene deposits, $\sigma$ is snowball-effect deposition constant, $D_{pt}$ is average pore throat diameter and $D_{ptcr}$ is critical pore throat diameter.

$$\gamma = \gamma_i\left(1 + \sigma V_{s2}^d\right) \text{ when } D_{pt} < D_{ptcr} \tag{10}$$

$$\gamma = 0 \text{ when } D_{pt} \geq D_{ptcr} \tag{11}$$

In the third term for entrainment 536, $\beta$ may be entrainment rate coefficient, ($v_0$) may be interstitial velocity, and ($v_{cr}$) may be critical interstitial velocity. The entrainment rate coefficient and/or other parameters may be user controlled parameter(s). The third term may model reversal of asphaltene deposition when the velocity of the flowing phase is fast enough for the asphaltene to peel away from the surface/pores. The third term may represent the entrainment of asphaltene deposits by the flowing phase when the interstitial velocity ($v_0$) is larger than a critical interstitial velocity ($v_{cr}$). The entrainment rate of the asphaltene deposits may be directly proportional to the amount of asphaltene deposit/fractional pore volume of asphaltene deposit ( $V_{s2}^d$ ) present in porous media, and the difference between the actual interstitial velocity and the critical interstitial velocity necessary for asphaltene deposit mobilization.

At step 540, production characteristics of the subsurface region may be modeled. The production characteristics of the subsurface region may include permeability damage 542 and viscosity change 544. Deposition of asphaltene may cause the permeability of the subsurface region to be reduced. Asphaltene deposits may occupy pore space and reduce the permeability of the subsurface region. Reduction in permeability of the subsurface region may include reduction in porosity of the subsurface region. The porosity of the subsurface region may be reduced by the amount of asphaltene deposit/fractional pore volume of asphaltene deposit ( $V_{s2}^d$ ) present in porous media. The permeability damage 542 may be governed by the below, where Ø is the resulting porosity and $\emptyset_0$, is the initial porosity:

$$\emptyset = \emptyset_0 - V_{s2}^d \qquad (12)$$

Modeling change in permeability may include modeling permeability resistance factors. For example, the permeability resistance factor ($R_f$) may be calculated as shown below, where b is an exponent factors (e.g., ranging from 3 to 7):

$$R_f = \left(\frac{\emptyset_0}{\emptyset}\right)^b \qquad (13)$$

Viscosity changes 544 may include change in viscosity of flowing phase (e.g., oil). The viscosity of flowing phase may change (e.g., become thicker) due to precipitated asphaltene (e.g., asphaltene precipitate 512) and/or flocculated asphaltene (e.g., asphaltene flocculate 522). The ratio of resulting viscosity to initial viscosity ( $\frac{\mu}{\mu_{po}}$ ) may be governed by the below, where $a$ is a contant (e.g., user-controlled constant, constant based on subsurface region properties) and $\mu_{po}$ is the pseudo oil viscosity.

$$\frac{\mu}{\mu_{po}} = 1 + a\left(C_{O,ppa} + C_{O,pfa}\right) \qquad (14)$$

The asphaltene simulation 500 shown in FIG. 5 may assume that the precipitated and flocculated asphaltene moves with flowing/oil phase at the same speed. Rather than modeling asphaltene precipitation and flocculation as chemical reaction processes, the asphaltene simulation 500 may model asphaltene precipitation and flocculation as physical changes in which the hydrocarbon components are packed differently (e.g., less tightly, more tightly). The amount of precipitated and flocculated asphaltene may go into the same material balance equation as the heaviest component (C_H2) in the oil phase. With the pseudo-component framework for asphaltene simulation, the volume of precipitated and flocculated asphaltene may be tracked by solving the volumetric concentration of the pseudo precipitated asphaltene (ppa) component and the pseudo flocculated asphaltene (pfa) component. In the asphaltene simulation 500, the deposited asphaltene may not be introduced to the fluid and there may be no new phase other than regular isothermal compositional cases. The valid phase states of the asphaltene simulation 500 may include gas-oil-water, oil-water, and gas-water. Cells in a subsurface representation for simulation of asphaltene may be assigned as being gas-oil-water, oil-water, or gas-water.

Performing the asphaltene simulation 500 may include solving material balance equations for light hydrocarbon component, heavy hydrocarbon component, and water component. In the material balance equations, $S_\alpha$ may represent phase saturation, $V$ may represent cell volume, and $\gamma_\alpha$ may represent phase mass density.

The material balance equation for light hydrocarbon component may be given as:

$$\frac{\delta}{\Delta t} V \emptyset \sum_{pc=po,pg} \rho_{pc} x_{pc,i} \sum_{\alpha=O,G} S_\alpha C_{\alpha,pc} + q_i$$
$$- \sum_k T_k \sum_{pc=po,pg} \rho_{pc} x_{pc,i} \left[ \sum_{\alpha=O,G} \frac{k_{r\alpha}}{\mu_\alpha R_f} C_{\alpha,pc} (\Delta p - \gamma_\alpha g \Delta h) \right] = R_i$$

The material balance equation for the heaviest hydrocarbon component may include precipitation, flocculation, and deposition of asphaltene. This single material balance equation may cover all pseudo components (po, ppa, pfa) for the asphaltene simulation 500. The material balance equation for the heaviest hydrocarbon component may be given as:

$$\frac{\delta}{\Delta t} V \varnothing \sum_{pc=po,pg,ppa,pfa} \rho_{pc} x_{pc,i} \sum_{\alpha=O,G} S_\alpha C_{\alpha,pc} + \frac{\delta}{\Delta t} V V_{s2} \rho_{ps2} + q_i$$

$$- \sum_k^{faces} T_k \sum_{pc=po,pg,ppa,pfa} \rho_{pc} x_{pc,i} \left[ \sum_{\alpha=O,G} \frac{k_{r\alpha}}{\mu_\alpha R_f} C_{\alpha,pc} (\Delta p - \gamma_\alpha g \Delta h) \right] = R_i$$

Water component may exist only in aqueous phase and the material balance equation for water component may be given as:

$$\frac{\delta}{\Delta t} V \varnothing \sum_{pc=pw} \rho_{pc} x_{pc,i} \sum_{\alpha=A} S_\alpha C_{\alpha,pc} + q_i - \sum_k^{faces} T_k \sum_{pc=pw} \rho_{pc} x_{pc,i} \left[ \sum_{\alpha=A} \frac{k_{r\alpha}}{\mu_\alpha R_f} C_{\alpha,pc} (\Delta p - \gamma_\alpha g \Delta h) \right]$$
$$= R_i$$

A material balance for the flocculated asphaltene component may be given as:

$$\frac{\delta}{\Delta t} \left[ V \varnothing \rho_{pfa} S_O C_{O,pfa} \right] + \frac{\delta}{\Delta t} \left[ V V_{s2}^d \rho_{s2} \right] + q_{pfa} - \sum_k^{faces} T_k \rho_{pfa} \left[ \frac{k_{rO}}{\mu_O R_f} C_{O,pfa} (\Delta p_O - \gamma_O g \Delta h) \right]$$
$$- V \varnothing S_o \left( k_{af} \cdot \rho_{ppa} \cdot C_{O,ppa} - k_{fa} \cdot \rho_{pfa} \cdot C_{O,pfa} \right) = R_{pfa}$$

Referring back to FIG. 1, the processor 11 may be configured to provide information processing capabilities in the system 10. As such, the processor 11 may comprise one or more of a digital processor, an analog processor, a digital circuit designed to process information, a central processing unit, a graphics processing unit, a microcontroller, an analog circuit designed to process information, a state machine, and/or other mechanisms for electronically processing information. The processor 11 may be configured to execute one or more machine-readable instructions 100 to facilitate asphaltene simulation. The machine-readable instructions 100 may include one or more computer program components. The machine-readable instructions 100 may include one or more of a subsurface representation component 102, a simulation component 104, a production characteristic component 106, an operation component 108, and/or other computer program components.

The subsurface representation component 102 may be configured to obtain subsurface representation information and/or other information. Obtaining subsurface representation information may include one or more of accessing, acquiring, analyzing, creating, determining, examining, generating, identifying, loading, locating, measuring, opening, receiving, retrieving, reviewing, selecting, storing, utilizing, and/or otherwise obtaining the subsurface representation information. The subsurface representation component 102 may obtain subsurface representation information from one or more locations. For example, the subsurface representation component 102 may obtain subsurface representation information from a storage location, such as the electronic storage 13, electronic storage of a device accessible via a network, and/or other locations. The subsurface representation component 102 may obtain subsurface representation information from one or more hardware components (e.g., a computing device, a component of a computing device) and/or one or more software components (e.g., software running on a computing device). Subsurface representation information may be stored within a single file or multiple files.

The subsurface representation information may define a subsurface representation. The subsurface representation information may define a subsurface representation by including information that describes, delineates, identifies, is associated with, quantifies, reflects, sets forth, and/or otherwise defines one or more of content, quality, attribute, feature, and/or other aspects of the subsurface representation. For example, the subsurface representation information may define a subsurface representation by including information that makes up the content of the subsurface representation and/or information that is used to identify/determine the content of the subsurface representation. Other types of subsurface representation information are contemplated.

A subsurface representation may refer to a computer-generated representation of a subsurface region, such as a one-dimensional, two-dimensional, and/or three-dimensional model of a subsurface region. A subsurface representation may be defined by and/or include the subsurface configurations simulated by one or more subsurface models. A subsurface model may refer to a computer model (e.g., program, tool, script, function, process, algorithm) that generates subsurface representations. A subsurface model may simulate subsurface configuration within a region underneath the surface (subsurface region). A subsurface model may simulate subsurface configurations by generating one or more subsurface representations.

A subsurface representation may be used as and/or may be referred to as a digital analog. A subsurface representation may include geologically plausible arrangement of rock obtained from a modeling process (e.g., stratigraphic forward modeling process). A subsurface representation may be representative of a subsurface region in the real world. A subsurface region may refer to a part of earth located beneath the surface/located underground. A subsurface region may refer to a part of earth that is not exposed at the surface. A subsurface region may be defined in a single dimension (e.g., a point, a line) or in multiple dimensions (e.g., a surface, a volume). A subsurface region may include and/or be part of a reservoir or a field.

A reservoir may refer to a location at which one or more resources are stored. For example, a reservoir may refer to a location at which hydrocarbons are stored. For instance, a reservoir may refer to a location including rocks in which oil and/or natural gas have accumulated. A reservoir may include regions below the surface. A reservoir may include one or more wells. For example, a reservoir may include one or more injection wells (e.g., for injection of fluid), one or more production wells (e.g., for extraction of oil or gas), and/or other wells. The term "subsurface region," "formation," "subsurface formation," "subterranean formation," "subsurface volume of interest," "subsurface region of interest," and the like may be utilized interchangeably with the term "reservoir."

A field may refer to an accumulation, pool, or group of pools of hydrocarbons or other mineral resources in a subsurface region. A hydrocarbon field may include a reservoir in a shape that traps hydrocarbons. A well may refer to a hole that is drilled in the subsurface. A well may be drilled in the subsurface for exploration and/or recovery of resources from the subsurface, such as hydrocarbons. For example, a well may be drilled for production of hydrocarbons (e.g., as a production well). The term "wellbore," "well bore," "borehole," and the like may be utilized interchangeably with the term "well." A well may be located offshore, or even deepwater, such as drilled into a conventional reservoir that is located below the seafloor. Embodiments may be utilized in scenarios where the hydrocarbon contains asphaltenes that may precipitate, flocculate, and/or deposit.

A subsurface representation may define subsurface configuration of a subsurface region. A subsurface representation may define subsurface configuration at different locations within a subsurface region. A subsurface representation may define (e.g., characterize, describe, identify, quantify, etc.) subsurface configuration of a subsurface region using values of one or more subsurface properties. The subsurface configuration in different portions of the subsurface representation may be defined by values of subsurface propert(ies) in those portions. For example, the subsurface representation may be made up of cells (e.g., voxels), and the cells may include and/or be associated with particular values of subsurface propert(ies). The cells of the subsurface representation may be used to convey information relating to the subsurface propert(ies) in the corresponding portions of the subsurface representation. For example, the cells of the subsurface representation may include and/or be associated with information on grain size, porosity, permeability, material, material contact (e.g., water-oil contact, gas-oil contact), and/or other subsurface properties to represent subsurface configuration in the corresponding portions of the subsurface representation.

Subsurface configuration may refer to attribute, quality, and/or characteristics of a subsurface region. Subsurface configuration may refer to physical arrangement of materials (e.g., subsurface elements) within a subsurface region. Examples of subsurface configuration simulated by a subsurface model may include types of subsurface materials, characteristics of subsurface materials, compositions of subsurface materials, arrangements/configurations of subsurface materials, physics of subsurface materials, and/or other subsurface configuration. For instance, subsurface configuration may include and/or define types, shapes, and/or properties of materials and/or layers that form subsurface (e.g., geological, petrophysical, geophysical, stratigraphic) structures.

A subsurface property may refer to property (e.g., characteristic, trait) of materials in a subsurface region. Examples of subsurface properties include flow velocity, grain size, grain type, grain lithology, material type, porosity, permeability, sediment concentration, water depth, and/or other properties of materials in a subsurface region. Subsurface properties may include one or more geological, petrophysical, geophysical, and/or stratigraphic properties.

In some implementations, the subsurface representation may define subsurface configuration of a real region. For example, the subsurface representation may define subsurface configuration of a physical subsurface region, such as a region in the real world (e.g., a real basin, a real reservoir). In some implementations, the subsurface representation may define simulated subsurface configuration of a simulated region. For example, the subsurface representation may define simulated subsurface configuration of a virtual subsurface region, such as a region in a simulation generated using one or more computer models.

The simulation component 104 may be configured to simulate asphaltene in a subsurface region. Simulation of asphaltene in a subsurface region may include simulation of asphaltene precipitation, asphaltene flocculation, and/or asphaltene deposition in the subsurface region. Simulation of asphaltene in a subsurface region may include modeling characteristics and/or changes in asphaltene in the subsurface region. Simulation of asphaltene in a subsurface region may include modeling characteristics and/or changes in the subsurface region due to asphaltene in the subsurface region. Simulation of asphaltene in a subsurface region may include creation of a computer simulation of static and/or dynamic characteristics of asphaltene in the subsurface region. Simulation of asphaltene in a subsurface region may include creation of a computer simulation of static and/or dynamic characteristics of the subsurface region due to asphaltene in the subsurface region.

Simulation of asphaltene in a subsurface region may include modeling of processes that occur in the subsurface region. For example, simulation of asphaltene in a subsurface region may include modeling of asphaltene precipitation, asphaltene flocculation, and/or asphaltene deposition in the subsurface region. For instance, simulation of asphaltene in a subsurface region may include modeling the precipitation of asphaltene from fluid in the subsurface region, the flocculation of asphaltene precipitates into larger particles, and deposition of the asphaltene flocculates in porous media. Simulation of asphaltene in a subsurface region may include modeling the impact of asphaltene in the subsurface region, such as pore throat plugging, permeability damage, and/or viscosity change for fluid phase in the subsurface region. Simulation of asphaltene in a subsurface region may include some or all of the processes described in/with respect to the flow diagram for asphaltene simulation 500 shown in FIG. 5. Other types of simulation of asphaltene in a subsurface region are contemplated.

Asphaltene in a subsurface region may be simulated based on the subsurface representation, a pseudo-component framework for asphaltene simulation, and/or other information. For example, the asphaltene precipitation, the asphaltene flocculation, and/or the asphaltene deposition in the subsurface region may be simulated based on the subsurface representation, a pseudo-component framework for asphaltene simulation, and/or other information. The asphaltene simulation for a subsurface region may be performed using the subsurface representation for the subsurface region. The asphaltene simulation for a subsurface region may be performed using information obtained from the subsurface representation for the subsurface region. For example, the subsurface representation may be divided into cells for different parts of the subsurface region, and the subsurface properties for the cells may be used to model behavior of asphaltene within the corresponding parts of the subsurface region.

Use of the pseudo-component framework for asphaltene simulation may include breaking down of components into pseudo components, such as described with respect to the listing of pseudo components 400 shown in FIG. 4. For example, the pseudo-component framework for asphaltene simulation may include multiple pseudo components in oil phase. The multiple pseudo components in oil phase may include a pseudo oil (po) component, a pseudo precipitated asphaltene (ppa) component, a pseudo flocculated asphaltene (pfa) component, and/or other components.

Use of the pseudo-component framework for asphaltene simulation may enable some of all of the processes described in/with respect to the flow diagram for asphaltene simulation 500 to be used. The pseudo-component framework for asphaltene simulation may treat the asphaltene precipitation, the asphaltene flocculation, and the asphaltene deposition as physical changes of a single component. The pseudo-component framework for asphaltene simulation may not treat the asphaltene precipitation, the asphaltene flocculation, and the asphaltene deposition as chemical changes. In some implementations, the pseudo-component framework for asphaltene simulation may reduce complexity of asphaltene simulation by defining mole fraction/concentration at pseudo-component level and not at phase level. The pseudo-component framework for asphaltene simulation may enable straightforward extension of the asphaltene simulation to an existing subsurface simulator (e.g., reservoir simulator).

In some implementations, the simulation of the asphaltene precipitation, the asphaltene flocculation, and the asphaltene deposition in the subsurface region may include determination of an amount of precipitated asphaltene, an amount of flocculated asphaltene, and/or an amount of deposited asphaltene in the subsurface region. Determining amount(s) of precipitated asphaltene, flocculated asphaltene, and/or deposited asphaltene in the subsurface region may include ascertaining, approximating, calculating, establishing, estimating, finding, identifying, obtaining, performing, quantifying, and/or otherwise determining the amount(s) of precipitated asphaltene, flocculated asphaltene, and/or deposited asphaltene in the subsurface region. Determining amount(s) of precipitated asphaltene, flocculated asphaltene, and/or deposited asphaltene in the subsurface region may include determining the amount(s) of precipitated asphaltene, flocculated asphaltene, and/or deposited asphaltene in the subsurface region at one or more moments in time and/or over one or more durations of time. Determining amount(s) of precipitated asphaltene, flocculated asphaltene, and/or deposited asphaltene in the subsurface region may include determining quantity (e.g., by volume, by mass) of precipitated asphaltene, flocculated asphaltene, and/or deposited asphaltene in the subsurface region.

In some implementations, the simulation of asphaltene precipitation in the subsurface region may be performed based on pressure, temperature, composition, fugacity, and/or other information. For example, the simulation of the asphaltene precipitation in the subsurface region may be performed based on processes and/or relationships described with respect to asphaltene precipitation 510 (shown in FIG. 5) and/or equation (4). For instance, the processes and/or relationships described with respect to equation (4) may be used to calculate the amount of precipitated asphaltene based on cell pressure, temperature, composition, fluid fugacity, and precipitated asphaltene fugacity. Other simulations of asphaltene precipitation are contemplated.

In some implementations, the simulation of asphaltene flocculation in the subsurface region may include forward rate formation of flocculated asphaltene from precipitated asphaltene and backward rate formation of the precipitated asphaltene from the flocculated asphaltene. For example, the simulation of the asphaltene flocculation in the subsurface region may be performed based on processes and/or relationships described with respect to asphaltene flocculation 520 (shown in FIG. 5) and/or equations (5)-(7). For instance, the processes and/or relationships described with respect to equations (5)-(7) may be used to determine forward aggregation and backward disaggregation of flocculated asphaltene, and determine the amount of flocculated asphaltene. Other simulations of asphaltene flocculation are contemplated.

In some implementations, the simulation of asphaltene deposition in the subsurface region may include surface deposition, pore throat plugging, and entrainment. For example, the simulation of the asphaltene deposition in the subsurface region may be performed based on processes and/or relationships described with respect to asphaltene deposition 530 (shown in FIG. 5) and/or equation (8). For instance, the processes and/or relationships described with respect to equation (8) may be used to determine asphaltene deposition via surface deposition, pore throat plugging, and entrainment. Other simulations of asphaltene deposition are contemplated.

The production characteristic component 106 may be configured to determine one or more production characteristics of a subsurface region. Determining production characteristic(s) of a subsurface region may include ascertaining, approximating, calculating, establishing, estimating, finding, identifying, obtaining, performing, quantifying, and/or otherwise determining the production characteristic(s) of the subsurface region. Determining the production characteristic(s) of the subsurface region may include determining types and/or values of the production characteristic(s) of the subsurface region.

The production characteristic(s) of the subsurface region may be determined based on the simulation of asphaltene in the subsurface region and/or other information. The production characteristic(s) of the subsurface region may be determined based on the simulation of asphaltene precipitation, asphaltene flocculation, and/or asphaltene deposition in the subsurface region and/or other information. The production characteristic(s) of the subsurface region may be output by the simulation of asphaltene in the subsurface region. The production characteristic(s) of the subsurface region may be determined based on information output by the simulation of asphaltene in the subsurface region. The production characteristic(s) of the subsurface region may be output by the simulation of asphaltene precipitation, asphaltene flocculation, and/or asphaltene deposition in the subsurface region. The production characteristic(s) of the subsurface region may be determined based on information output by the simulation of asphaltene precipitation, asphaltene flocculation, and/or asphaltene deposition in the subsurface region. Other determinations of the production characteristic(s) of the subsurface region are contemplated.

Production characteristics of a subsurface region may refer to attribute, quality, configuration, parameter, and/or characteristics of matter produced, extracted, recovered, and/or used from/in the subsurface region. Production characteristics of a subsurface region may refer to characteristics of matter inside the subsurface region. Production characteristics of a subsurface region may refer to characteristics of the subsurface region that control, affect, reflect, and/or otherwise determine production from the subsurface region. Production characteristics of a subsurface region may refer to characteristics of the subsurface region that control, affect, reflect, and/or otherwise determine how materials are produced in/from the subsurface region. Production characteristics of a subsurface region may static characteristics (that do not change with time/operation) and/or dynamic characteristics (that changes with time/operation). For example, production characteristics of a subsurface region may include permeability of rock and/or viscosity of flowing phase (e.g., oil) in the subsurface region. Other types of production characteristics are contemplated.

In some implementations, determination of the production characteristic(s) of the subsurface region may include determination of permeability damage and/or viscosity reduction due to asphaltene in the subsurface region. For example, permeability damage in the subsurface region may be determined based on processes and/or relationships described with respect to permeability damage 542 (shown in FIG. 5) and/or equation (13). For instance, the processes and/or relationships described with respect to equation (13) may be used to calculate permeability damage due to asphaltene deposition. As another example, viscosity reduction in the subsurface region may be determined based on processes and/or relationships described with respect to viscosity change 544 (shown in FIG. 5) and/or equation (14). For instance, the processes and/or relationships described with respect to equation (14) may be used to calculate viscosity reduction due to asphaltene precipitation and flocculation.

The operation component 108 may be configured to facilitate one or more operations for a subsurface region. Facilitating an operation for a subsurface region may include assisting, automating, carrying out, controlling, designing, enabling, implementing, initiating, performing, planning, scheduling, setting up, and/or otherwise facilitating the operation for a subsurface region. The operation(s) for a subsurface region may be facilitated based on the production characteristic(s) of the subsurface region and/or other information. For example, the operation(s) for a subsurface region may be facilitated based permeability damage and/or viscosity reduction due to asphaltene in the subsurface region. The operation(s) for a subsurface region may be facilitated based on other production characteristic(s) of the subsurface region.

An operation for a subsurface region may refer to an operation relating to a subsurface region. An operation for a subsurface region may refer to a performance of work on and/or use of the subsurface region. An operation for a subsurface region may refer to an activity involving the subsurface region. An operation for a subsurface region may be performed at/in the subsurface region or away from the subsurface region.

For example, facilitating operation(s) for a subsurface region may include provision (e.g., visual presentation, audible playback, transfer) of information relating to the production characteristic(s) and/or the facilitating operation(s) for the subsurface region. Facilitating operation(s) for a subsurface region may include providing estimated/simulated values of asphaltene precipitation, asphaltene flocculation, and/or asphaltene deposition in the subsurface region and/or estimated/simulated production characteristic(s) of the subsurface region to one or more computing devices and/or one or more operators for use in operation(s) for the subsurface region. Facilitating operation(s) for a subsurface region may include using production characteristic(s) and/or simulation of asphaltene in the subsurface region to design and/or implement operations of equipment (e.g., operations of wells, operations of fluid facilities with chemical agents, such as one or more solvents, to address the asphaltene) to reduce, minimize, or eliminate asphaltene precipitation, asphaltene flocculation, and/or asphaltene deposition in the subsurface region. Facilitating operation(s) for a subsurface region may include using production characteristic(s) and/or simulation of asphaltene in the subsurface region to design and/or implement operations of equipment (e.g., operations of wells, operations of fluid facilities with chemical agents, such as one or more solvents, to address the asphaltene) to reduce, minimize, or eliminate impact of asphaltene to the subsurface region. For example, the production characteristic(s) and/or simulation of asphaltene in the subsurface region may be used to reduce, minimize, or eliminate formation damage or well clogging. The production characteristic(s) and/or simulation of asphaltene in the subsurface region may be used to simulate/test operations to dissolve asphaltene the subsurface region. Other facilitations of operations for the subsurface region are contemplated.

Implementations of the disclosure may be made in hardware, firmware, software, or any suitable combination thereof. Aspects of the disclosure may be implemented as instructions stored on a machine-readable medium, which may be read and executed by one or more processors. A machine-readable medium may include any mechanism for storing or transmitting information in a form readable by a machine (e.g., a computing device). For example, a non-transitory, tangible computer-readable storage medium may include read-only memory, random access memory, magnetic disk storage media, optical storage media, flash memory devices, and others, and a machine-readable transmission media may include forms of propagated signals, such as carrier waves, infrared signals, digital signals, and others. Firmware, software, routines, or instructions may be described herein in terms of specific exemplary aspects and implementations of the disclosure, and performing certain actions.

In some implementations, some or all of the functionalities attributed herein to the system 10 may be provided by external resources not included in the system 10. External resources may include hosts/sources of information, computing, and/or processing and/or other providers of information, computing, and/or processing outside of the system 10.

Although the processor 11, the electronic storage 13, and the electronic display 14 are shown to be connected to the interface 12 in FIG. 1, any communication medium may be used to facilitate interaction between any components of the system 10. One or more components of the system 10 may communicate with each other through hardwired communication, wireless communication, or both. For example, one or more components of the system 10 may communicate with each other through a network. For example, the processor 11 may wirelessly communicate with the electronic storage 13. By way of non-limiting example, wireless communication may include one or more of radio communication, Bluetooth communication, Wi-Fi communication, cellular communication, infrared communication, or other wireless communication. Other types of communications are contemplated by the present disclosure.

Although the processor 11, the electronic storage 13, and the electronic display 14 are shown in FIG. 1 as single entities, this is for illustrative purposes only. One or more of the components of the system 10 may be contained within a single device or across multiple devices. For instance, the processor 11 may comprise a plurality of processing units. These processing units may be physically located within the same device, or the processor 11 may represent processing functionality of a plurality of devices operating in coordination. The processor 11 may be separate from and/or be part of one or more components of the system 10. The processor 11 may be configured to execute one or more components by software; hardware; firmware; some combination of software, hardware, and/or firmware; and/or other mechanisms for configuring processing capabilities on the processor 11.

It should be appreciated that although computer program components are illustrated in FIG. 1 as being co-located within a single processing unit, one or more of computer program components may be located remotely from the other computer program components. While computer program components are described as performing or being configured to perform operations, computer program components may comprise instructions which may program processor 11 and/or system 10 to perform the operation.

While computer program components are described herein as being implemented via processor 11 through machine-readable instructions 100, this is merely for ease of reference and is not meant to be limiting. In some implementations, one or more functions of computer program components described herein may be implemented via hardware (e.g., dedicated chip, field-programmable gate array) rather than software. One or more functions of computer program components described herein may be software-implemented, hardware-implemented, or software and hardware-implemented.

The description of the functionality provided by the different computer program components described herein is for illustrative purposes, and is not intended to be limiting, as any of computer program components may provide more or less functionality than is described. For example, one or more of computer program components may be eliminated, and some or all of its functionality may be provided by other computer program components. As another example, processor 11 may be configured to execute one or more additional computer program components that may perform some or all of the functionality attributed to one or more of computer program components described herein.

The electronic storage media of the electronic storage 13 may be provided integrally (i.e., substantially non-removable) with one or more components of the system 10 and/or as removable storage that is connectable to one or more components of the system 10 via, for example, a port (e.g., a USB port, a Firewire port, *etc.)* or a drive (e.g., a disk drive, *etc.).* The electronic storage 13 may include one or more of optically readable storage media (e.g., optical disks, *etc.),* magnetically readable storage media (e.g., magnetic tape, magnetic hard drive, floppy drive, *etc.),* electrical charge-based storage media (*e.g.,* EPROM, EEPROM, RAM, *etc.),* solid-state storage media (*e.g.,* flash drive, *etc.),* and/or other electronically readable storage media. The electronic storage 13 may be a separate component within the system 10, or the electronic storage 13 may be provided integrally with one or more other components of the system 10 (*e.g.,* the processor 11). Although the electronic storage 13 is shown in FIG. 1 as a single entity, this is for illustrative purposes only. In some implementations, the electronic storage 13 may comprise a plurality of storage units. These storage units may be physically located within the same device, or the electronic storage 13 may represent storage functionality of a plurality of devices operating in coordination.

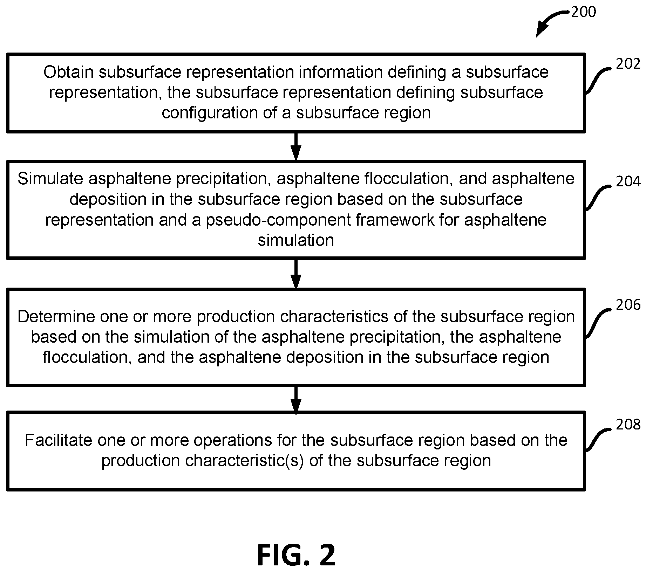

FIG. 2 illustrates a method 200 for performing asphaltene simulation using a pseudo-component framework. The operations of method 200 presented below are intended to be illustrative. In some implementations, method 200 may be accomplished with one or more additional operations not described, and/or without one or more of the operations discussed. In some implementations, two or more of the operations may occur substantially simultaneously.

In some implementations, method 200 may be implemented in one or more processing devices (*e.g.,* a digital processor, an analog processor, a digital circuit designed to process information, a central processing unit, a graphics processing unit, a microcontroller, an analog circuit designed to process information, a state machine, and/or other mechanisms for electronically processing information). The one or more processing devices may include one or more devices executing some or all of the operations of method 200 in response to instructions stored electronically on one or more electronic storage media. The one or more processing devices may include one or more devices configured through hardware, firmware, and/or software to be specifically designed for execution of one or more of the operations of method 200.

Referring to FIG. 2 and method 200, at operation 202, subsurface representation information and/or other information may be obtained. The subsurface representation information may define a subsurface representation. The subsurface representation may define subsurface configuration of a subsurface region. In some implementations, operation 202 may be performed by a processor component the same as or similar to the subsurface representation component 102 (Shown in FIG. 1 and described herein).

At operation 204, asphaltene precipitation, asphaltene flocculation, and asphaltene deposition in the subsurface region may be simulated based on the subsurface representation, a pseudo-component framework for asphaltene simulation, and/or other information. The pseudo-component framework for asphaltene simulation may treat the asphaltene precipitation, the asphaltene flocculation, and the asphaltene deposition as physical changes of a single component. In some implementations, operation 204 may be performed by a processor component the same as or similar to the simulation component 104 (Shown in FIG. 1 and described herein).

At operation 206, one or more production characteristics of the subsurface region may be determined based on the simulation of the asphaltene precipitation, the asphaltene flocculation, and the asphaltene deposition in the subsurface region, and/or other information. In some implementations, operation 206 may be performed by a processor component the same as or similar to the production characteristic component 106 (Shown in FIG. 1 and described herein).

At operation 208, one or more operations for the subsurface region may be facilitated based on the production characteristic(s) of the subsurface region and/or other information. In some implementations, operation 208 may be performed by a processor component the same as or similar to the operation component 108 (Shown in FIG. 1 and described herein).

In any of the above aspects, the various features may be implemented in hardware, or as software modules running on one or more processors/computers.

The invention also provides a computer program or a computer program product comprising instructions which, when executed by a computer, cause the computer to carry out any of the methods/method steps described herein, and a non-transitory computer-readable medium comprising instructions which, when executed by a computer, cause the computer to carry out any of the methods/method steps described herein. A computer program embodying the invention may be stored on a non-transitory computer-readable medium, or it could, for example, be in the form of a signal such as a downloadable data signal provided from an Internet website, or it could be in any other form.

**Claims**

1. A computer-implemented method (200) for asphaltene simulation, the method comprising:

    obtaining (202) subsurface representation information defining a subsurface representation, the subsurface representation defining subsurface configuration of a subsurface region;
    simulating (204) asphaltene precipitation, asphaltene flocculation, and asphaltene deposition in the subsurface region based on the subsurface representation and a pseudo-component framework for asphaltene simulation, wherein the pseudo-component framework for asphaltene simulation includes multiple pseudo components in oil phase, the multiple pseudo components in oil phase including a pseudo oil component (po), a pseudo precipitated asphaltene component (ppa), and a pseudo flocculated asphaltene component (pfa), the pseudo-component framework for asphaltene simulation treating the asphaltene precipitation, the asphaltene flocculation, and the asphaltene deposition as physical changes of a component by requiring sum of volume of the pseudo oil component (po), volume of the pseudo precipitated asphaltene component (ppa), and volume the pseudo flocculated asphaltene component (pfa) to equal to volume of the oil phase, the pseudo-component framework for asphaltene simulation not treating the asphaltene precipitation, the asphaltene flocculation, and the asphaltene deposition as chemical changes, wherein the oil phase is divided among the pseudo oil component (po), the pseudo precipitated asphaltene component (ppa), and the pseudo flocculated asphaltene component (pfa) as different physical forms of a heavy pure component, further wherein the asphaltene precipitation, the asphaltene flocculation, and the asphaltene deposition in the subsurface region are simulated by solving a single material balance equation for heaviest hydrocarbon component to cover the pseudo oil component (po), the pseudo precipitated asphaltene component (ppa), and the pseudo flocculated asphaltene component (pfa) for the asphaltene simulation;
    determining (206) one or more production characteristics of the subsurface region based on the simulation of the asphaltene precipitation, the asphaltene flocculation, and the asphaltene deposition in the subsurface region; and
    facilitating (208) one or more operations for the subsurface region based on the one or more production characteristics of the subsurface region.

2. The method of claim 1, wherein the simulation of the asphaltene precipitation, the asphaltene flocculation, and the asphaltene deposition in the subsurface region includes determination of an amount of precipitated asphaltene, an amount of flocculated asphaltene, and an amount of deposited asphaltene in the subsurface region.

3. The method of claim 1 or 2, wherein the simulation of the asphaltene precipitation in the subsurface region is performed based on pressure, temperature, composition, and fugacity.

4. The method of claim 1, 2 or 3, wherein the simulation of the asphaltene flocculation in the subsurface region includes forward rate formation of flocculated asphaltene from precipitated asphaltene and backward rate formation of the precipitated asphaltene from the flocculated asphaltene.

5. The method of any of the preceding claims, wherein the simulation of the asphaltene deposition in the subsurface region includes surface deposition, pore throat plugging, and entrainment.

6. The method of any of the preceding claims, wherein the pseudo-component framework for asphaltene simulation reduces complexity of asphaltene simulation by defining mole fraction/concentration at pseudo-component level and not at phase level.

7. The method of any of the preceding claims, wherein the one or more production characteristics of the subsurface region include permeability of rock and/or viscosity of oil in the subsurface region.

8. The method of any of the preceding claims, wherein the determination of the one or more production characteristics of the subsurface region includes determination of permeability damage and/or viscosity reduction due to asphaltene in the subsurface region.

9. A system (10) for asphaltene simulation, the system comprising:

one or more physical processors (11) configured by machine-readable instructions (100) to carry out the method of any of the preceding claims.

10. One or more computer programs comprising instructions (100) which, when executed by computer, cause the computer to carry out the method of any of claims 1 to 8.

11. A non-transitory computer-readable storage medium having the one or more programs of claim 10 stored thereon.

**Patentansprüche**

1. Computerimplementiertes Verfahren (200) zur Asphaltensimulation, das Verfahren Folgendes umfassend:

Erhalten (202) von Untergrunddarstellungsinformationen, die eine Untergrunddarstellung definieren, wobei die Untergrunddarstellung eine Untergrundkonfiguration eines Untergrundbereichs definiert;
Simulieren (204) von Asphaltenfällung, Asphaltenflockung und Asphaltenabscheidung in dem Untergrundbereich basierend auf der Untergrunddarstellung und einem Pseudokomponentenrahmenwerk zur Asphaltensimulation, wobei das Pseudokomponentenrahmenwerk zur Asphaltensimulation mehrere Pseudokomponenten in Ölphase beinhaltet, wobei die mehreren Pseudokomponenten in Ölphase eine Pseudoölkomponente (po), eine pseudogefällte Asphaltenkomponente (ppa) und eine pseudogeflockte Asphaltenkomponente (pfa) beinhalten, das Pseudokomponentenrahmenwerk zur Asphaltensimulation die Asphaltenfällung, die Asphaltenflockung und die Asphaltenabscheidung als physikalische Veränderungen einer Komponente behandelt, indem erfordert wird, dass eine Summe des Volumens der Pseudoölkomponente (po), des Volumens der pseudogefällten Asphaltenkomponente (ppa) und des Volumens der pseudogeflockten Asphaltenkomponente (pfa) gleich dem Volumen der Ölphase ist, das Pseudokomponentenrahmenwerk zur Asphaltensimulation die Asphaltenfällung, die Asphaltenflockung und die Asphaltenabscheidung nicht als chemische Veränderungen behandelt, wobei die Ölphase unter der Pseudoölkomponente (po), der pseudogefällten Asphaltenkomponente (ppa) und der pseudogeflockten Asphaltenkomponente (pfa) als unterschiedliche physikalische Formen einer schweren reinen Komponente aufgeteilt ist, wobei ferner die Asphaltenfällung, die Asphaltenflockung und die Asphaltenabscheidung in dem Untergrundbereich durch Lösen einer einzelnen Materialbilanzgleichung für eine schwerste Kohlenwasserstoffkomponente simuliert werden, um die Pseudoölkomponente, die pseudogefällte Asphaltenkomponente (ppa) und die pseudogeflockte Asphaltenkomponente (pfa) für die Asphaltensimulation abzudecken;
Bestimmen (206) einer oder mehrerer Produktionscharakteristiken des Untergrundbereichs basierend auf der Simulation der Asphaltenfällung, der Asphaltenflockung und der Asphaltenabscheidung in dem Untergrundbereich; und
Erleichtern (208) einer oder mehrerer Operationen für den Untergrundbereich basierend auf der einen oder den mehreren Produktionscharakteristiken des Untergrundbereichs.

2. Verfahren nach Anspruch 1, wobei die Simulation der Asphaltenfällung, der Asphaltenflockung und der Asphaltenabscheidung in dem Untergrundbereich eine Bestimmung einer Menge an gefälltem Asphalten, einer Menge an geflocktem Asphalten und einer Menge an abgeschiedenem Asphalten in dem Untergrundbereich beinhaltet.

3. Verfahren nach Anspruch 1 oder 2, wobei die Simulation der Asphaltenfällung in dem Untergrundbereich basierend auf Druck, Temperatur, Zusammensetzung und Fugazität durchgeführt wird.

4. Verfahren nach Anspruch 1, 2 oder 3, wobei die Simulation der Asphaltenflockung in dem Untergrundbereich eine Vorwärtsgeschwindigkeit einer Bildung von geflocktem Asphalten aus gefälltem Asphalten und eine Rückwärtsgeschwindigkeit einer Bildung des gefällten Asphaltens aus dem geflocktem Asphalten beinhaltet.

5. Verfahren nach einem der vorhergehenden Ansprüche, wobei die Simulation der Asphaltenabscheidung in dem Untergrundbereich Oberflächenabscheidung, Porenhalsverstopfung und Mitreißen beinhaltet.

6. Verfahren nach einem der vorhergehenden Ansprüche, wobei das Pseudokomponentenrahmenwerk zur Asphaltensimulation eine Komplexität der Asphaltensimulation reduziert, indem Molfraktion/Konzentration auf Pseudokomponentenebene und nicht auf Phasenebene definiert wird.

7. Verfahren nach einem der vorhergehenden Ansprüche, wobei die eine oder die mehreren Produktionscharakteristiken des Untergrundbereichs Permeabilität von Gestein und/oder Viskosität von Öl in dem Untergrundbereich beinhalten.

8. Verfahren nach einem der vorhergehenden Ansprüche, wobei die Bestimmung der einen oder der mehreren Produktionscharakteristiken des Untergrundbereichs eine Bestimmung von Permeabilitätsschädigung und/oder einer Viskositätsreduktion aufgrund von Asphalten in dem Untergrundbereich beinhaltet.

9. System (10) zur Asphaltensimulation, das System Folgendes umfassend:
einen oder mehrere physische Prozessoren (11), die durch maschinenlesbare Anweisungen (100) konfiguriert sind, um das Verfahren nach einem der vorhergehenden Ansprüche durchzuführen.

10. Ein oder mehrere Computerprogramme, die Anweisungen (100) umfassen, die, wenn sie vom Computer ausgeführt werden, den Computer veranlassen, das Verfahren nach einem der Ansprüche 1 bis 8 durchzuführen.

11. Nichtflüchtiges computerlesbares Speichermedium mit einem oder mehreren auf ihm gespeicherten Programmen nach Anspruch 10.


**Revendications**

1. Procédé (200) mis en œuvre par ordinateur pour la simulation d'asphaltène, le procédé comprenant :

   l'obtention (202) d'informations de représentation souterraine définissant une représentation souterraine, la représentation souterraine définissant la configuration souterraine d'une zone souterraine ;
   la simulation (204) de précipitation d'asphaltène, de floculation d'asphaltène et de dépôt d'asphaltène dans la zone souterraine sur la base de la représentation souterraine et d'un cadre pseudo-composant pour une simulation d'asphaltène, le cadre pseudo-composant pour une simulation d'asphaltène comprenant plusieurs pseudo-composants en phase huileuse, les plusieurs pseudos-composants en phase huileuse comprenant un pseudo composant huile (po), un pseudo-composant asphaltène précipité (ppa) et un pseudo composant asphaltène floculé (pfa), le cadre pseudo-composant pour la simulation d'asphaltène traitant la précipitation d'asphaltène, la floculation d'asphaltène et le dépôt d'asphaltène comme changements physiques d'un composant en requérant la somme du volume du pseudo composant huile (po), du volume du pseudo composant asphaltène précipité (ppa) et du volume du pseudo composant asphaltène floculé (pfa) pour égaler le volume de phase huile, le cadre pseudo-composant pour la simulation d'asphaltène ne traitant pas la précipitation d'asphaltène, la floculation d'asphaltène, et le dépôt d'asphaltène sous forme de changements chimiques, dans lequel la phase huileuse est divisée entre le pseudo composant huile (po), le pseudo composant asphaltène précipité (ppa) et le pseudo composant asphaltène floculé (pfa) sous forme de différentes formes physiques d'un composant pur lourd, en outre la précipitation d'asphaltène, la floculation d'asphaltène et le dépôt d'asphaltène dans la zone souterraine sont simulés en résolvant une équation de bilan de matière pour le composant hydrocarbure le plus lourd pour couvrir le pseudo composant huile (po), le pseudo composant asphaltène précipités (ppa), et le pseudo composant asphaltène floculé (pfa) pour la simulation d'asphaltène ;
   la détermination (206) d'une ou plusieurs caractéristiques de production de la zone souterraine en fonction de la simulation de la précipitation d'asphaltène, de la floculation d'asphaltène et du dépôt d'asphaltène dans la zone souterraine ; et
   la facilitation (208) d'une ou plusieurs opérations pour la zone souterraine sur la base de la ou des caractéristiques de production de la zone souterraine.

2. Procédé selon la revendication 1, dans lequel la simulation de la précipitation d'asphaltène, de la floculation d'asphaltène et du dépôt d'asphaltène dans la zone souterraine comprend la détermination d'une quantité d'asphaltène précipité, d'une quantité d'asphaltène floculé et d'une quantité d'asphaltène déposé dans la zone souterraine.

3. Procédé selon la revendication 1 ou 2, dans lequel la simulation de la précipitation d'asphaltène dans la zone souterraine est effectuée sur la base de la pression, de la température, de la composition et de la fugacité.

4. Procédé selon la revendication 1, 2 ou 3, dans lequel la simulation de la floculation de l'asphaltène dans la zone souterraine comprend la formation à vitesse directe d'asphaltène floculé à partir d'asphaltène précipité et la formation à vitesse inverse de l'asphaltène précipité à partir de l'asphaltène floculé.

**5.** Procédé selon l'une quelconque des revendications précédentes, dans lequel la simulation du dépôt d'asphaltène dans la zone souterraine comprend un dépôt de surface, un colmatage de gorge de pore et un entraînement.

**6.** Procédé selon l'une quelconque des revendications précédentes, dans lequel le cadre de pseudo-composant pour la simulation d'asphaltène réduit la complexité de la simulation d'asphaltène en définissant une fraction/concentration molaire au niveau du pseudo-composant et non au niveau de la phase.

**7.** Procédé selon l'une quelconque des revendications précédentes, dans lequel la ou les caractéristiques de production de la zone souterraine comprennent la perméabilité de la roche et/ou la viscosité de l'huile dans la zone souterraine.

**8.** Procédé selon l'une quelconque des revendications précédentes, dans lequel la détermination de la ou des caractéristiques de production de la zone souterraine comprend la détermination de l'endommagement de perméabilité et/ou de la réduction de viscosité due à l'asphaltène dans la zone souterraine.

**9.** Système (10) de simulation d'asphaltène, le système comprenant :
un ou plusieurs processeurs physiques (11) configuré par des instructions lisibles par machine (100) pour réaliser le procédé selon l'une quelconque des revendications précédentes.

**10.** Programme(s) informatique(s) comprenant des instructions (100) qui, lorsqu'il(s) est/sont exécuté(s) par un ordinateur, amènent l'ordinateur à exécuter le procédé selon l'une quelconque des revendications 1 à 8.

**11.** Support de stockage non transitoire lisible par ordinateur ayant le ou les programmes selon la revendication 10 stockés sur celui-ci.

_—10

Processor 11

Machine-Readable Instructions <u>100</u>

Subsurface Representation Component 102

Simulation Component 104

Production Characteristic Component 106

Operation Component 108

Interface <u>12</u>

Electronic Storage <u>13</u>

Electronic Display <u>14</u>

# FIG. 1

200

Obtain subsurface representation information defining a subsurface representation, the subsurface representation defining subsurface configuration of a subsurface region
202

Simulate asphaltene precipitation, asphaltene flocculation, and asphaltene deposition in the subsurface region based on the subsurface representation and a pseudo-component framework for asphaltene simulation
204

Determine one or more production characteristics of the subsurface region based on the simulation of the asphaltene precipitation, the asphaltene flocculation, and the asphaltene deposition in the subsurface region
206

Facilitate one or more operations for the subsurface region based on the production characteristic(s) of the subsurface region
208

# FIG. 2

Precipitated Asphaltene 310

Flocculated Asphaltene 320

Deposited Asphaltene 330

Surface 300

# FIG. 3

400

| Phase | Pseudo Components | Pure Components |
|-------|-------------------|-----------------|
| Gas | pg | C_L |
| | | C_H1 |
| | | C_H2 |
| Oil | po | C_L |
| | | C_H1 |
| | | C_H2 |
| | ppa | C_H2 |
| | pfa | C_H2 |
| Water | pw | Water |

# FIG. 4

**FIG. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KHANIFAR AHMAD et al.** Modelling of Asphaltene Precipitation and Deposition during WAG Application. *International Petroleum Technology Conference*, 07 February 2012

- **KHANIFAR AHMAD et al.** Study of asphaltene precipitation and deposition phenomenon. *National Postgraduate Conference*, 2011